(19) Europäisches Patentamt
European Patent Office
Office européen des brevets

(11) Publication number: 0 227 604
A2

(12) # EUROPEAN PATENT APPLICATION

(21) Application number: 86810601.4

(22) Date of filing: 18.12.86

(51) Int. Cl.[4]: C 07 K 7/08
A 61 K 37/02

(30) Priority: 23.12.85 AU 3729/85

(43) Date of publication of application:
01.07.87 Bulletin 87/27

(84) Designated Contracting States:
AT BE CH DE ES FR GB GR IT LI LU NL SE

(71) Applicant: SANDOZ AG
Lichtstrasse 35
CH-4002 Basel (CH)

(84) Designated Contracting States:
BE CH ES FR GB GR IT LI LU NL SE

(71) Applicant: SANDOZ-PATENT-GMBH
Humboldtstrasse 3
D-7850 Lörrach (DE)

(84) Designated Contracting States: DE

(71) Applicant: SANDOZ-ERFINDUNGEN
Verwaltungsgesellschaft m.b.H.
Brunner Strasse 59
A-1235 Wien (AT)

(84) Designated Contracting States: AT

(72) Inventor: Werner, Gudrun
Franz Grassler Gasse 8-10 Haus B
A-1238 Vienna (AT)

McCray, Joseph Willis, Dr.
Schoberstrasse 53/1
A-1130 Vienna (AT)

(54) **Use of oligopeptides in the treatment of viral infections.**

(57) Oligopeptides having variable length and sequences from the receptor-binding region of viruses which use specific cellular receptors for the penetration of host cells.

EP 0 227 604 A2

**Description**

USE OF OLIGOPEPTIDES IN THE TREATMENT OF VIRAL INFECTIONS

Although the common cold is a usually harmless illness. secondary infections can nevertheless have serious consequences. Common cold can be caused by a number of different viruses and microorganisms whereby rhino viruses play an important role. It is known that a protective immunity against a particular serotype can be induced by live or inactivated viruses. The greatest problem in the development of a vaccine against rhino viruses is the large number (ca. !2O) of serotypes as the immunity induced in this way is serotype specific. In recent years the possibility has been discovered of obtaining antibodies against parts of the antigenic protein which are normally not recognised by the immune system by employing short synthetic peptides optionally linked to carrier proteins. Vaccines comprising such peptides for example against foot and mouth disease (FMD) and E. coli enterotoxin are already in an advanced stage of development.

Oligopeptides having variable lengths with sequences from the receptor-binding regions of viruses which employ a specific cellular receptor for penetration of the host cell induce, optionally after coupling to a suitable carrier protein, on local or systemic application neutralising antibodies, which on infection by the natural antigen (= complete virus particle) are not increasingly formed. The three-dimensional structure of Rhino I4 and Polio I virus particles has been clarified by X-ray scattering. Regions have been identified which are binding sites for cellular receptors and which consist of deep tucks or "canyons" on the surface of the virus particle. It has been stated in the literature that these receptor-binding sites are not accessible to anti-bodies for steric reasons. It has however become apparent that antibodies obtained using oligopeptides do in fact neutralise the virus particles, i.e. that they are suitable for the treatment or prophylaxis of infections caused by such viruses. In a particular embodiment according to the invention oligopeptides are used which have partial sequences from the VPI and VP3 envelope proteins of Rhino I4 which lie at the base of the tuck and are in part also conserved in other Picorna viruses. Oligopeptides having these sequences have induced antibodies in test animals, which were positive in an in vitro neutralisation test.

Preferably oligopeptides are used having sequences from the receptor-binding region of picorna viruses PVPR and having the general formula

$NH_2$-$X_{1-5}$-Tyr-$X_6$-Pro-Pro-Gly-Ala-$X_7$-$X_8$-Pro-$X_{9-14}$-COOH
(His) (Ile) (Lys)
(Val)

wherein $X_{1-14}$ stand independently for any amino acid whereby one or more of the amino acids $X_{1-5}$ or $X_{9-14}$ may be replaced by a direct bond.

Particularly suitable according to the invention are oligopeptides with conserved sequences from the capsid proteins VP3 and VPI having the formula PVP3

$NH_2$-$X'_{1-5}$-Tyr-$X'_6$-Pro-Pro-Gly-Ala-$X'_7$-$X'_8$-Pro-$X'_{9-11}$-Cys-COOH
(Ile)
(Val) PVP3

wherein $X'_1$ stands for a basic amino acid such as Lys, His or Arg $X'_2$ stands for an aliphatic or aromatic amino acid such as Leu, Val, Ile, Ala, Tyr, Phe or Trp, $X'_3$ stands for any amino acid. $X'_4$ stands for an aliphatic amino acid such as Leu, Val, Ile, Ala, Thr, Tyr, or Ser, $X'_5$ stands for Ala, Ser, Thr, Val, Ile or Leu, $X'_6$ stands for Thr, Ala, Ile, Ser, Leu or Val and $X'_7$ -$X'_{11}$ stand independently for any amino acid, or having the formula PVPI

$NH_2$-$X''_1$-$X''_2$-Gln-$X''_4$-Met-Tyr-Val-Pro-Pro-Gly-Ala-Pro-$X''_8$-Pro-$X''_9$-$X''_{10}$-Cys-COOH
(His) (Lys) (Lys) PVP1

wherein $X''_1$ and $X''_2$ are the same or different and each represent any amino acid. $X''_4$ stands for Ala, Ile, Tyr, Val, Leu, Phe or Trp. $X''_8$ and $X''_9$ are the same or different and each represent a charged amino acid such as Lys, His, Arg, Asp, Asn, Glu or Gln and $X''_{10}$ stands for any amino acid whereby one or more of the amino acids defined by $X'_1$ to $X'_5$, $X'_9$ to $X'_{11}$ or $X''_1$, $X''_2$, $X''_9$ or $X''_{10}$ may be replaced by a direct bond.

Especially suitable according to the invention are oligopeptides with partial acid sequences from the receptor-binding region of the Rhino-I4 virus and having the formula

$NH_2$-Val-Val-Gln-Ala-Met-Tyr-Val-Pro-Pro-Gly-Ala-Pro-Asn-Pro-Lys-Glu-Cys-COOH

PVPla

or

$NH_2$-Lys-Leu-Ile-Leu-Ala-Tyr-Thr-Pro-Pro-Gly-Ala-Arg-Gly-Pro-Gln-Asp-Cys-COOH

PVP3a.

The oligopeptides according to the invention may be prepared e.g. by conventional solid phase peptide synthesis preferably on automatic or semi-automatic peptide synthesizers. The amino acids in protected form are successively coupled. Following introduction of the final amino acid the oligopeptide is cleaved from the resin and deprotected. The final product is purified and analysed in conventional manner, e.g. employing high pressure liquid chromatography.

These oligopeptides can also be used in dimeric or polymeric form. A monomeric oligopeptide containing a Cys can for example be oxidised into the dimer.

Oligopeptides of variable lengths with sequences from the receptor-binding regions of viruses can be chemically coupled to carrier proteins, e.g. keyhole-limpet hemocyamin (KLH) or BSA, to form immunogenic conjugates. For immunisation in humans the carrier must be physiologically acceptable. Preferably e.g. carriers will be used which themselves act as vaccines e.g. tetanus toxoid and/or diptheria toxoid.

The oligopeptides according to the invention are particularly useful for immunisation in particular against rhino viruses. This can be achieved by administering an effective amount of an oligopeptide according to the invention as such or in dimeric or polymeric form, or linked to a physiologically acceptable carrier protein.

Prophylactic protection can for example be induced by repeated local application to the mucous membrane of the nose. Prophylaxis can also be achieved e.g. by intramuscular application e.g. of IOO ug to I mg of the oligopeptide in question. The oligopeptides can however also be used for the therapeutic treatment of existing infections.

In particular oligopeptides are employed with partial amino acid sequences from picorna virus envelope proteins which are involved in the binding of virus particles on cellular receptors. The peptides contain sequences from the capsid proteins VPI and VP3 of Rhino virus type I4. Parts of these peptides are however conserved for various other picorna viruses such as Rhino, Polio or FMD and can therefore induce neutralising antibodies against other Rhino-Serotypes and Polio and FMD-viruses or other Picorna viruses.

The oligopeptides according to the invention can for example be employed with pharmaceutically acceptable diluents or carriers and e.g. additionally with a natural or synthetic detergent for local e.g. nasal application. Therapeutic preparations can contain one or more oligopeptides or dimers, polymers or conjugates thereof in the form of cocktails. They can also contain other antigens in physical admixture. Preparations for nasal administration can contain e.g. O.I to O.5 mg/ml of oligopeptide.

The following examples illustrate the invention without in any way limiting the scope thereof. Temperatures are given in degrees centigrade.

**EXAMPLE I** : Preparation of the heptadecapeptide
$H_2N$-Val-Val-Gln-Ala-Met-Tyr-Val-Pro-Pro-Gly-Ala-Pro-Asn-Pro-Lys-Glu-Cys-COOH (PVPla)

The peptide is prepared stepwise employing solid phase-peptide synthesis on a p-alkoxybenzylalcohol polystyrene resin (I% DVB cross-linked).

The coupling of the first I6 acids proceeds in each case via the FMOC-amino acid derivative whereby the following amino acids are provided with a side chain protecting group as indicated)

- Cys (acetamidomethyl-)-
- Glu (γ-tert.-butylester)-
- Lys (e-amino-BOC)-
- Tyr (tert.-butylether)-.

**a)** Coupling of Val to H-I6-p-Alkoxybenzylalcoholresin

The following reagents were reacted in a semi-automatic peptide synthesizer (SP 640 - Labortec) for 90 min. at 2O C in DMF as a solvent: I. eq. -H-I6-p-alkoxybenzylalcohol-resin

3. eq. BOC-Val-OH
3. eq. hydroxybenzotriazole (HOBT)
3.3. eq. dicyclohexylcarbodiimide (DCCI)

All excess reactants and side products are removed by filtration and washing.

The preceding coupling steps are carried out in an analogous manner.

**b)** Separation of peptide from resin with simultaneous removal of acid labile protecting groups

The peptide coupled resin is shaken for 9O min. at 2O C with 55% trifluoroacetic acid in methylenechloride. The peptide is separated from the resin by filtration and the residual resin washed through twice with 2O% trifluoroacetic acid in methylenechloride and once with methylenechloride alone. IO% anisol is added to the separation solutions as t-butyl cation scavenger. The peptide filtrate is evaporated at 35 C and the residue reprecipitated from ether, washed three times with ether and dried in vacuum.

c) Removal of the -Cys(acetamidomethyl)-protecting group

The peptide is dissolved in 80% aq. trifluoroacetic acid, kept free of oxygen with nitrogen gas and reduced to Cys-peptide mercaptide with 1.2 eq. Hg(II)-acetate for 15 min. at 0 . Liberation of peptide from mercaptide is carried out using $H_2S$. The resulting difficultly soluble Hg sulfide is completely removed by filtration and washed through with 80% acetic acid ($O_2$-free). The peptide filtrate is evaporated at 35  and directly lyophilised from water ($O_2$-free).

d) Preparative HPLC-purification of the peptide

Carrier : RP C 18. 15-20 microns 300 A (silica gel)

Buffers : A 2.8 g $NaClO_4$

2 ml $H_3PO_4$ + $H_2O$ 2 litre solution

B 2.8 g $NaClO_4$

2 ml $H_3PO_4$

1200 ml acetonitrile + $H_2O$ 2 litre solution.

Gradient system

100% A, 0.1% TFA - 60 min. - 100% B

(0.1% TFA/$CH_3CN$ 40/60)

Pure fractions are combined and lyophilised. The pure peptide is then freed from the peptide-TFA-salt form by strongly basic ion-exchange (acetate-form).

The resulting peptide had a MW of 1800.14 g/mol.

Aminoacid analysis.

Val 2.88 (3.00) Glu 1.95(2.00) Ala 1.98(2.00)

Met 0.84 (4.00) Gly 0.98(1.00) Tyr 0.92(1.00)

Pro 3.90 (4.00) Asp 0.96(1.00) Lys 1.00(1.00)

Cys 0.93 (1.00)

Other peptides according to the invention and in particular PVP3a can be prepared in an analogous manner.

**EXAMPLE 2** : Neutralisation of Rhino 14 viruses with antibodies against synthetic Peptides

1. Peptides employed

PVP1a and PVP3a as hereinbefore illustrated.

2. Chemical coupling of the peptides to KLH.

To a solution of 10 mg of KLH in 0.9 ml of sodium phosphate buffer are added 0.1 ml 50mM 3-maleicimidobenzoic acid-N-hydroxy-succinimide ester (MBS) in dimethylformamide and the mixture stirred for 30 minutes at room temperature. A further 0.05 ml of MBS solution are then added and stirring continued for a further 30 minutes. The cloudiness is centrifuged off and the clear solution chromatographed at 4° over a Sephadex G25-column (elution with phosphate buffer, pH 7.5). The eluate is measured photometrically and the fractions having the highest protein content combined.

10 mg of peptide are dissolved at pH 6.5 in 1 ml of 0.1M phosphate buffer/1mM ethylenediaminetetracetate (EDTA) (optionally with up to 20% acetonitrile). 1 ml of the peptide solution and 1ml of the reacted KLH-solution are combined, the pH value ajusted to 8.5 and the solution saturated with nitrogen. The mixture is incubated for 2 hours at room temperature and then dialysed against deficient PBS.

3. Immunisation of test animals

The conjugate solution obtained e.g. as described under 2 above is adjusted with deficient PBS to a protein concentration of 0.4 mg/ml. This solution is mixed 1:1 with complete Freund's adjuvant and injected intradermally into New Zealand White Rabbits (1ml per rabbit). After 10-14 days the animals are injected a second time subcutaneously with the same amount of conjugate mixed this time with incomplete Freund's adjuvant. After 7 and 14 days serum is taken from the animals for antibody purifaction. The antibody level against the peptide is determined by an ELISA.

4. Purification of the antibodies

4.1 Preparation of the affinity column.

5 ml of Affigel-102 are washed with 300 ml of cold distilled water, equilibrated with 0.1M phosphate buffer and sucked off. 100mg of maleicimido-β-alanyl-succinimide ester are dissolved in methanol/dimethylformamide (2/1) and the gel is then suspended therein and shaken for 2 hours at room temperature. The gel is then washed on a suction filter with PBS and resuspended in a solution of 10mg peptide in 10ml of 0.1M phosphate (pH6.5) with 0.1mM EDTA The pH value is adjusted to 8.0 with 1N NaOH, the suspension shaken for 1 hour at R.T. and then washed alternately with PBS and 0.1M glycine HCl/0.15M NaCl (pH 2.8) (total 2 litres). The gel is stored suspended in PBS with 0.03% sodium azide at 4°.

4.2 Affinity chromatography

5ml of anti-serum, diluted with an equal volume of PBS are very slowly applied to a small column (2,5-3ml prepared with a gel as described under 4.I) equilibrated with PBS. The column is washed through with PBS until no further protein was detectable in the eluate (extinction at 28Onm = O). The bound antibodies are eluted with O.IM glycine HCl/O.I5M NaCl (pH 2.8), whereby each fraction is added to 25u 2M Tris per ml of fraction volume. The fractions containing antibody are determined photometrically (extinction at 28Onm), combined and dialysed against PBS.

5. Neutralisation test

In cell-culture microtiter plates O.I ml of various purified antipeptide antisera (prepared e.g. as described under 4.2) prediluted (I:2) in a combination of Eagles MEM with 2% FCS, 4OnM Mg $Cl_2$ and I% penicillin/Streptomycin (Medium A) are diluted out in half rows with the same medium. Controls contain either a I : IO pre-diluted neutralising rabbit antiserum against complete virus particles (positive control) or medium A alone (virus control). To each well is added O.O5ml of viral broth (4.5 × $IO^5$ pfu/ml; pre-diluted I:IO with medium A) and incubated for I hour at 34° in 5% humid $CO_2$-atmosphere. O.5ml of a freshly prepared HELA-Ohio cell suspension ($IO^5$ cell/ml) are then added to each well and incubation continued for 48 hours under the same conditions. Medium is removed by shaking off and the cells remaining in the wells stained with crystal violet (2.5 g crystal violet, dissolved in I6.5ml of ethanol and 8Oml of formamide and filled to 26Oml with dist. water). Anti-PVPIa- and anti-PVP3a-antibodies neutralised Rhino I4 infection up to an end-dilution of I : 32. At the same time the cells in the virus control wells are completely lysed (no staining). Antibodies against peptides having completely different structures prepared in the same manner did not neutralise the infection.

**EXAMPLE 3**

Neutralisation of various serotypes of human rhino virus with antibodies against synthetic peptides

The preparation of the antibodies and the neutralisation test are carried out as described in Example 2. Various serotypes of human rhino viruses, polio I and 2 viruses, echo 9 virus and Coxcackie B virus are employed in the neutralisation test. The results of which are given in the following table.

| Activity (dilution factor) | Serotype (rhino) or virus tested |
|---|---|
| none | 1a,2,4,7,8,13,15,18,19,20,21,22,25,33,38,45,47, 62,75,85,Polio 1, Polio 2, Echo 9,Coxsackie B 3 |
| weak ( 1:8) | 9,10,16,40,45,68,71 |
| medium (1:8-1:32) | 17,24,26,28,32,36,51,58,72 |
| strong ( 1:32) | 3,5,6,14,23,27,35,37,48,50,55,64 |

The viruses underlined belong to a group of rhino viruses which use a different receptor for cell penetration.

From these results it can be seen that ca. 6O% of the serotypes investigated are neutralised, 25% equally well or better and 33% less strongly than Rhino I4.

This clearly shows that the antibodies neutralise, and can therefore protect against a large group of human rhino virus serotypes.

**EXAMPLE 4**

Local intranasal immunisation with a dimeric oligopeptide in detergent solution

I. Preparation of the dimer of PVP3a

To a solution of 4 mg of PVP3a oligopeptide in 2 ml of sterile O.IM sodium phosphate buffer (pH = 8.O) are added 2Oul of 3O% $H_2O_2$ with stirring at R.T. which is continued for 2 hours. By this time more than 98% of the free sulfhydryl groups are oxidised (Ellmann's reagent). In order to facilitate the taking up of the dimer through the nasal epithelium. Triton x-IOO. a non-ionic detergent is added at a concentration of O.I%.

2. Intranasal immunisation

The dimer preparation thus obtained is diluted to a concentration of 0.2mg/ml with PBS containing 0.1% Triton x-100. Each of two rabbits receives in total 1 ml (0.2mg). 0.5 ml in each nostril, applied with a plastic syringe (without needle).

Two control animals receive the same amount of 0.1% Triton x-100 in PBS without peptide dimer. This treatment is repeated every 6 to 8 days and blood samples taken every 6 to 8 days to prepare serum for determination of anti-peptide antibody content. Three weeks following commencement of the treatment a large increase in anti-peptide antibodies is seen in rabbits immunised with peptide dimer.

3. Neutralisation test

With the help of an affinity column the anti-peptide antibodies contained in the serum are purified (cf. Example 1.4) and show the same results as given in the table of Example 3.

## Claims

1. Oligopeptides having variable length and sequences from the receptor-binding region of viruses which use specific, cellular receptors for the penetration of host cells.

2. An oligopeptide according to Claim 1 having the formula

$NH_2$-$X_{1-5}$-Tyr-$X_6$-Pro-Pro-Gly-Ala-$X_7$-$X_8$-Pro-$X_{9-14}$-COOH
(His) (Ile) (Lys)
(Val)

wherein $X_{1-14}$ stand independently for any amino acid whereby one or more of the amino acids $X_{1-5}$B or $X_{9-14}$ may be replaced by a direct bond.

3. An oligopeptide according to Claim 2 having the formula PVP3

$NH_2$-$X'_{1-5}$-Tyr-$X'_6$-Pro-Pro-Gly-Ala-$X'_7$-$X'_8$-Pro-$X'_{9-11}$-Cys-COOH
(Ile)
(Val) PVP3

or PVP1

$NH_2$-$X''_1$-$X''_2$-Cln-$X''_4$-Met-Tyr-Val-Pro-Pro-Gly-Ala-Pro-$X''_8$-Pro-$X''_9$-$X''_{10}$-Cys-COOH
(His) (Lys) (Lys)

wherein $X'_1$ stands for a basic amino acid, $X'_2$ stands for an aliphatic or aromatic amino acid, $X'_4$ stands for an aliphatic amino acid, $X'_5$ stands for Ala, Ser, Thr, Val, Ile or Leu, $X'_6$ stands for Thr, Ala, Ile, Ser, Leu or Val and $X'_3$ and $X'_7$-$X'_{11}$ independently stand for any amino acid; and $X''_4$ stands for Ala, Ile, Tyr, Val, Leu, Phe or Trp, $X''_4$ stands for Ala, Ile, Tyr, Val, Leu, Phe or Trp, $X''_8$ and $X''_9$ are the same or different and each represent a charged amino acid and $X''_1$. $X''_2$ and $X''_{10}$ are the same or different and each represent any amino acid, whereby one or more of the amino acids defined by $X'_1$ to $X'_5$, $X'_9$ to $X'_{11}$ or $X''_1$, $X''_2$, $X''_9$ or $X''_{10}$ may be replaced by a direct bond.

4. An oligopeptide according to Claim 3 wherein $X'_1$, stands for Lys, His or Arg, $X'_2$ stands for Leu, Val, Ile, Ala, Tyr, Phe or Trp, $X'_4$ stands for Leu, Val, Ile, Ala, Thr, Tyr or Ser, $X''_8$ and $X''_9$ are the same or different and each represent Lys, His, Arg, Asp, Asn, Glu or Gln and the remaining substituents are as defined in Claim 3, whereby one or more of the amino acids defined by $X'_1$ to $X'_5$, $X'_9$ to $X'_{11}$ or $X''_1$, $X''_2$, $X''_9$ or $X''_{10}$ may be replaced by a di bond.

5. An oligopeptide according to Claim 3 selected from

$NH_2$-Val-Val-Gln-Ala-Met-Tyr-Val-Pro-Pro-Gly-Ala-Pro-Asn-Pro-Lys-Glu-Cys-COOH

or PVP1a

$NH_2$-Lys-Leu-Ile-Leu-Ala-Tyr-Thr-Pro-Pro-Gly-Ala-Arg-Gly-Pro-Gln-Asp-Cys-COOH

PVP3a.

6. An oligopeptide according to any one of Claims I to 5 in dimeric or polymeric form.
7. An oligopeptide according to any one of Claims I to 5 conjugated to a carrier protein.
8. A pharmaceutical composition comprising an oligopeptide according to any one of Claims I to 6 optionally conjugated to a physiologically acceptable carrier protein together with a pharmaceutically acceptable diluent or carrier.
9. A pharmaceutical composition according to Claim 8 comprising an oligopeptide according to any one of Claims I to 6 optionally conjugated to a physiologically acceptable carrier protein together with a pharmaceutically acceptable, natural or synthetic detergent in a form suitable for nasal administration.
IO. An oligopeptide according to any one of Claims I to 6 optionally conjugated to a physiologically acceptable carrier protein for use in the therapeutic or prophylactic treatment of viral infections.
II. An oligopeptide according to any one of Claims I to 6 optionally conjugated to a physiologically acceptable carrier protein for use in immunisation against viral infections.
I2. A oligopeptide for use according to Claim IO or II whereby the viral infections are caused by rhino and other picorna viruses.